# EUROPEAN PATENT APPLICATION

(11) **EP 4 283 631 A1**
(43) Date of publication of application: **29.11.2023**
(21) Application number: 22742612.9
(22) Date of filing: 19.01.2022
(51) Int. Cl.: G16H 50/50, G16H 30/40

(54) **PREDICTION SYSTEM, CONTROL METHOD, AND CONTROL PROGRAM**

(30) Priority: 20.01.2021 JP 2021007477
(71) Applicant: Kyocera Corporation, Kyoto-shi Kyoto 612-8501 (JP)
(72) Inventor: WATANABE, Kenichi, Kyoto-shi, Kyoto 612-8501 (JP); KYOMOTO, Masayuki, Kyoto-shi, Kyoto 612-8501 (JP); HASHIDA, Masahiko, Kyoto-shi, Kyoto 612-8501 (JP); HONDA, Shintaro, Kyoto-shi, Kyoto 612-8501 (JP); WADA, Naoya, Kyoto-shi, Kyoto 612-8501 (JP)
(74) Representative: TBK
(86) International application number: PCT/JP2022/001798
(87) International publication number: WO 2022/158490

(57) **Abstract**

A prediction image is generated and output that represents a condition of a target region of a subject. A prediction system includes a prediction information acquirer that acquires (a) a subject image representing a target region of a subject at a first time, and (b) first prediction information regarding the target region at a second time when a predetermined period has elapsed from the first time; and a prediction image generation unit that generates a prediction image indicating a condition of the target region at the second time from the subject image based on the first prediction information and output the prediction image.

## Description

### TECHNICAL FIELD

The present disclosure relates to a prediction system, a control method, and a control program for predicting the condition of a target region of a human body.

### BACKGROUND OF INVENTION

As described in Patent Document 1, assistance in diagnosing osteoporosis by using a neural network has been devised.

### CITATION LIST

### PATENT LITERATURE

Patent Document 1: JP 2008-36068 A

### SUMMARY

According to an aspect of the present disclosure, a prediction system includes a prediction information acquirer that acquires (a) a subject image representing a target region of a subject at a first time and (b) first prediction information regarding the target region at a second time when a predetermined period has elapsed from the first time; and a prediction image generation unit that generates a prediction image from the first prediction information and the subject image by predicting a condition of the target region at the second time and output the prediction image.

According to an aspect of the present disclosure, a control method for a prediction system includes acquiring (a) a subject image representing a target region of a subject at a first time and (b) first prediction information regarding the target region at a second time when a predetermined period has elapsed from the first time; and generating a prediction image from the first prediction information and the subject image by predicting a condition of the target region at the second time and outputting the prediction image. The prediction system includes a prediction image generation model that can generate the prediction image by using the subject image and the first prediction information.

According to each aspect of the present disclosure, a prediction system may be implemented by a computer. In this case, the scope of the present disclosure also includes a control program of the prediction system that causes a computer to implement the prediction system by causing the computer to operate as each unit (software element) included in the prediction system, and a computer-readable recording medium recording the control program.

When the prediction system is implemented by a plurality of computers, the prediction system may be implemented by the computers by operating each computer as each unit (software element) included in each of the plurality of computers constituting the prediction system.

### BRIEF DESCRIPTION OF THE DRAWINGS

FIG. 1 is a block diagram illustrating a configuration example of a prediction system according to an aspect of the present disclosure.
FIG. 2 is a block diagram illustrating a configuration example of a prediction system according to another aspect of the present disclosure.
FIG. 3 is a block diagram illustrating an example of a configuration of a prediction system according to an aspect of the present disclosure.
FIG. 4 is a diagram illustrating an example of a configuration of a neural network included in a prediction image generation unit.
FIG. 5 is a flowchart illustrating an example of a process flow performed by a prediction system according to a first embodiment.
FIG. 6 is a block diagram illustrating an example of a configuration of a prediction system according to another aspect of the present disclosure.
FIG. 7 is a diagram illustrating an example of a configuration of a neural network included in the prediction information generation unit.
FIG. 8 is a flowchart illustrating an example of a process flow performed by a prediction system according to a second embodiment.
FIG. 9 is a block diagram illustrating an example of a configuration of a prediction system according to another aspect of the present disclosure.
FIG. 10 is a flowchart illustrating an example of a flow of training processing on a neural network included in the prediction information generation unit.
FIG. 11 is a flowchart illustrating another example of the process flow performed by the prediction system according to the second embodiment.
FIG. 12 is a block diagram illustrating an example of a configuration of a prediction system according to another aspect of the present disclosure.
FIG. 13 is a diagram illustrating an example of a configuration of a neural network included in a prediction image generation unit.
FIG. 14 is a flowchart illustrating an example of a flow of training processing on a neural network included in an intervention effect prediction unit.
FIG. 15 is a flowchart illustrating an example of a process flow performed by a prediction system according to a third embodiment.

### DESCRIPTION OF EMBODIMENTS

In order to improve the condition of a target region of a subject or slow down the onset and progression of a disorder, intervention such as surgery and lifestyle guidance is desirably started as early as possible.

In order to start the intervention at an early stage, it is important to make the subject recognize a prediction result for the condition of a target region of the subject and to make the subject understand the necessity of the intervention.

### First Embodiment

### Overview of Prediction System

A prediction system according to an aspect of the present disclosure is a system that generates a prediction image by predicting a change in the condition of a target region of a body of a subject and outputs the prediction image. Here, the target region may be any region of the body of the subject, and may be, for example, any one of the whole body, the head, the eyes, the mouth, the neck, the arms, the hands, the trunk, the waist, the buttocks, the legs, and the feet. The prediction image may be an image obtained by predicting a change in the condition of any one of skin, hair, an eyeball, a tooth, a gum, a muscle, fat, a bone, cartilage, a j oint, an intervertebral disc, and the like in the target region.

The prediction image may be an image obtained by predicting a change occurring in the target region of the subject affected by a disorder. For example, the prediction image may be an image indicating the shapes (for example, an abdominal girth, a chest girth, a height, swelling, atrophy, a joint angle, and a curvature) and/or the appearances (for example, a posture, wrinkles, spots, redness, turbidity, darkening, and yellowing) of the target region of the subject.

In one example, the subject may have a disorder at the target region of the subject. In this case, the prediction image may be an image obtained by predicting a change in the symptom in the target region of the subject which change is caused by the effect of the disorder. In the present specification, a prediction system that generates and outputs a prediction image will be described as an example, the prediction image being generated by predicting a change in the symptom in the target region of the subject. In this case, the prediction image includes an image indicating the effect of the disorder of the subject on the target region. Here, the "image indicating the effect on the target region" may be any image representing a change in the shape of the target region affected by the disorder, a qualitative or quantitative change caused in the tissue of the target region by the disorder, or the like.

The disorder may include at least one selected from the group consisting of obesity, alopecia, cataracts, periodontal disease, rheumatoid arthritis, Heberden's node, hallux valgus, osteoarthritis, spondylosis deformans, compression fracture and sarcopenia. The disorder may include (i) a syndrome such as metabolic syndrome and locomotive syndrome, which represents a collective pathology formed by a group of diverse symptoms, and (ii) a physical change such as aging and tooth alignment.

Based on a subject image representing the target region of the subject at the first time, the prediction system generates a prediction image by predicting the symptom in the target region at a second time when a predetermined period has elapsed from the first time. As used herein, the term "subject image" may refer to image data representing a subject image.

Here, the first time may be, for example, a time when a subject image corresponding to a captured image of the target region of the subject is acquired. The first time may typically be a time when a subject image representing a captured image of the current condition of the target region of the subject is acquired. That is, the first point in time may be intended to be substantially the present point in time. The predetermined period may be any period of time from the first time, and may be 6 months, 1 year, 5 years, 10 years, or 50 years. That is, the second point in time may be intended to be substantially any point in time in the future. The predetermined period is not limited to one period and may include a plurality of periods. That is, the prediction image may include images generated by predicting the symptom of the target region of the subject at a plurality of times such as 6 months, 1 year, 5 years, 10 years, and 50 years after the first time.

The subject image includes an image representing the target region of the subject at the first time. In one example, the subject image may be a captured appearance image of one of the whole body, the head, the upper body, the lower body, the upper limb, and the lower limb of the subject. In another example, the subject image may be a captured medical image of the target region of the subject which is used to examine the subject. Hence, the medical image may include at least one selected from the group consisting of an X-ray image, a computed tomography (CT) image, a magnetic resonance imaging (MRI) image, a positron emission tomography (PET) image, and an ultrasonic image of the subject corresponding to a captured image of the subject. In another example, the subject image may be an image indicating the shapes (for example, the abdominal girth, chest girth, height, swelling, atrophy, joint angle, and curvature) and/or the appearances (for example, the posture, wrinkles, spots, redness, turbidity, darkening, and yellowing) of the target region of the subject.

The prediction system uses the first prediction information regarding the target region at the second time in addition to the subject image in order to generate the prediction image.

The first prediction information may be information regarding the symptom of the target region of the subject at the second time. In this case, the first prediction information may include information indicating the symptom of the target region of the subject at a plurality of times, such as 6 months, 1 year, 5 years, 10 years, and 50 years after the first time. The first prediction information is, for example, information including a prediction regarding a symptom likely to occur in the target region of the subject and the period of time when the symptom is likely to occur, and the degree of progression of the symptom of the target region of the subject.

The first prediction information may be information regarding the shapes and/or appearances of the target region of the subject at the second time. In this case, the information may be information indicating the shapes (for example, the abdominal girth, chest girth, and height) and/or the appearances (for example, the posture, wrinkles, and spots) of the target region of the subject at a plurality of times such as 6 months, 1 year, 5 years, 10 years, and 50 years after the first time.

The first prediction information may be information regarding the shapes and/or appearances of the target region, which are related to the disorder of the target region. The first prediction information may include information listed below as information that is likely to change in the target region of the subject. The first prediction information may include, for example, (i) information regarding obesity such as body weight, body mass index (BMI), abdominal girth, visceral fat mass, blood pressure, blood glucose level, lipids, uric acid level, and liver function level, (ii) information regarding alopecia such as the number of hairs, sex hormone level, Norwood scale, and Ludwig scale, (iii) information regarding cataracts such as visual acuity, vision breadth, the level of clouding, and Emery-Little scale, (iv) information regarding periodontal disease such as the degree of pain and the degree of swelling, the number of remaining teeth, gingival index, and periodontal pocket depth, (v) information regarding rheumatoid arthritis such as the degree of pain, the degree of swelling, joint angle, joint movable range, Larsen scale, and Stein blocker scale (vi) information regarding Heberden's node such as the degree of pain, the degree of swelling, and joint movable range, (vii) information regarding hallux valgus such as the degree of pain, the degree of swelling, joint movable range, HV angle, and M1-M2 angle, (viii) information regarding osteoarthritis such as the degree of pain, the degree of swelling, joint angle, joint movable range, the level of stiffness, joint cartilage thickness, Kellgren-Laurence (K-L) scale, and the presence of claudication (ix) information regarding spondylosis deformans such as the degree of pain, the degree of spinal curvature, spinal motion range, and K-L scale, (x) information regarding compression fracture such as the degree of pain and spinal motion range, and (xi) information regarding sarcopenia such as muscle mass, walking speed, and grip strength.

In this manner, based on the subject image representing the target region of the subject at the first time and the first prediction information regarding the target region at the second time when the predetermined period has elapsed from the first time, the prediction system generates a prediction image by predicting the condition of the target region at the second time, and outputs the prediction image.

The prediction system can output the condition of the target region at the second time as a visually easy-to-understand prediction image. The prediction image is generated from the subject image which is an image of the subject in question, and thus the prediction image is a real image which is convincing to the subject. Accordingly, for example, when a doctor or the like in charge of the subject presents the prediction image to the subject, the subject can recognize the condition of the target region at the second time, and the subject can easily understand the necessity of intervention.

The prediction image may be an image imitating a captured appearance image of one of the whole body, the head, the upper body, the lower body, the upper limb, and the lower limb of the subject. In another example, the prediction image may be an image imitating a captured medical image of the target region of the subject, which is obtained in the course of medical examination of the subject. In another example, the prediction image may be an image indicating the shapes (for example, the abdominal girth, chest girth, height, swelling, atrophy, joint angle, and curvature) and/or the appearances (for example, the posture, wrinkles, spots, redness, turbidity, darkening, and yellowing) of the target region of the subject. For example, when the subject image is an appearance image indicating the current skin appearance of the subject (for example, wrinkles, spots, redness, turbidity, darkening, and yellowing) and the first prediction information is information regarding the future degree of wrinkle, spot, redness, turbidity, darkening, or yellowing scale of the subject, the prediction system can output an image indicating the future skin appearance of the subject as the prediction image based on the subject image and the first prediction information. For example, when the subject image is a medical image indicating the current angle of a joint of the subject and the first prediction information is information regarding the future angle of the joint of the subject, the prediction system can output the following two images as prediction images: (1) a medical image indicating the current angle of the joint of the subject and a medical image indicating the future angle of the joint of the subject based on the first prediction information can be output as the prediction image; and (2) an appearance image indicating the current appearance of the joint of the subject and an image indicating the future appearance of the joint of the subject based on the first prediction information can be output as the prediction image.

### Configuration of Prediction System 100

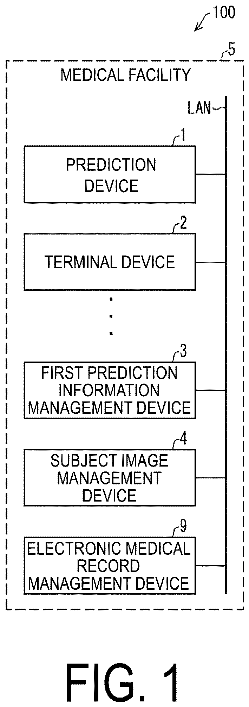

With reference to FIG. 1, a configuration of a prediction system 100 will be described that includes a prediction device 1 that acquires a subject image and first prediction information, generates a prediction image from the subject image based on the first prediction information, and outputs the prediction image. In an embodiment of the present disclosure, the prediction device 1 of the prediction system 100 can independently function as the above-described prediction system. FIG. 1 is a block diagram illustrating a configuration example of the prediction system 100 in a medical facility 5 in which the prediction device 1 is introduced.

As illustrated in FIG. 1, the prediction system 100 includes the prediction device 1 and one or more terminal devices 2 communicably connected to the prediction device 1. As described above, the prediction system 100 may include the prediction device 1 and a device (for example, the terminal device 2) that can present a prediction image output from the prediction device 1.

The prediction device 1 is a computer that acquires a subject image and first prediction information, generates a prediction image from the subject image based on the first prediction information, outputs the prediction image, and transmits the prediction image to the terminal device 2. As illustrated in FIG. 1, the prediction device 1 may be connected to a LAN of the medical facility 5. A configuration of the prediction device 1 will be described below.

The terminal device 2 receives a prediction image from the prediction device 1 and presents the prediction image. The terminal device 2 may be a computer used by medical personnel such as a doctor belonging to the medical facility 5, or the like. As illustrated in FIG. 1, the terminal device 2 may be connected to a LAN of the medical facility 5. The terminal device 2 may be, for example, a personal computer, a tablet terminal, or a smartphone. The terminal device 2 includes a communicator for transmitting and receiving data to and from another device, an input unit such as a keyboard and a microphone, and a display that can display prediction images.

In the prediction system 100, the prediction device 1 and the terminal device 2 are provided as separate bodies, but the prediction device 1 and the terminal device 2 may be integrated. For example, the prediction device 1 may have the function of the terminal device 2 by including a display unit that can display prediction images.

The prediction system 100 may further include a first prediction information management device 3, a subject image management device 4, and an electronic medical record management device 9.

The first prediction information management device 3 is a computer that functions as a server for managing first prediction information. As illustrated in FIG. 1, the first prediction information management device 3 may be connected to the LAN of the medical facility 5. In this case, the prediction device 1 may acquire the first prediction information of the subject from the first prediction information management device 3.

The subject image management device 4 is a computer that functions as a server for managing subject images. The subject image management device 4 may be a captured image of a subject who is examined about the condition of a target region in the medical facility 5. In one example, the subject image may be a medical image captured in the medical facility 5. For example, the subject image management device 4 may be communicably connected to an imaging device such as an X-ray imaging device in the medical facility 5. In this case, images captured by the imaging device may be recorded in the subject image management device 4 via, for example, the LAN. As illustrated in FIG. 1, the subject image management device 4 may be connected to the LAN of the medical facility 5. In this case, the prediction device 1 may acquire subject images from the subject image management device 4.

The electronic medical record management device 9 is a computer that functions as a server for managing electronic medical record information of a subject who is examined in the medical facility 5. As illustrated in FIG. 1, the electronic medical record management device 9 may be connected to the LAN of the medical facility 5. In this case, the prediction device 1 may acquire, from the electronic medical record management device 9, basic information related to the subject. The basic information is information included in the electronic medical record information and may include at least one selected from the group consisting of sex, age, height, weight of the subject, and information indicating the condition of the target region of the subject at the first time.

FIG. 1 illustrates an example in which the local area network (LAN) is disposed in the medical facility 5 and connects to the prediction device 1, the terminal device 2, the first prediction information management device 3, the subject image management device 4, and the electronic medical record management device 9, but the present disclosure is not limited to this configuration. The applicable network in the medical facility 5 may be, for example, the Internet, a telephone communication line network, an optical fiber communication network, a cable communication network, and a satellite communication network. The LAN in the medical facility 5 may be communicably connected to an external communication network. In this case the terminal device 2 may be, for example, a computer used by a patient.

In the prediction system 100, the prediction device 1 may be directly connected to at least one of the terminal device 2, the first prediction information management device 3, the subject image management device 4, and the electronic medical record management device 9 without using the LAN. The terminal device 2, the first prediction information management device 3, the subject image management device 4, and the electronic medical record management device 9 that can communicate with the prediction device 1 may be provided in plural. Furthermore, in the prediction system 100, a plurality of the prediction devices 1 may be introduced.

### Configuration of Prediction System 100a

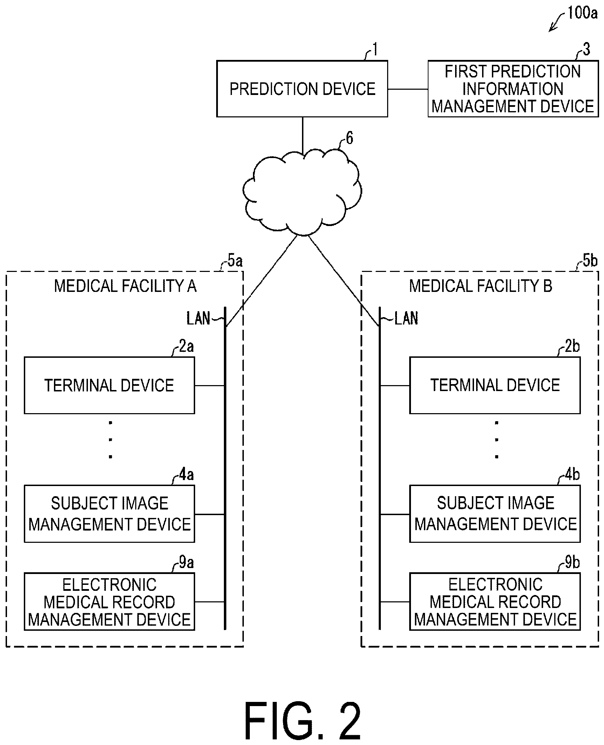

The prediction device 1 may be communicably connected to LANs each disposed in a respective one of a plurality of medical facilities 5 via a communication network 6 instead of a computer installed in a predetermined medical facility 5. FIG. 2 is a block diagram illustrating a configuration example of a prediction system 100a according to another aspect of the present disclosure.

The prediction system 100a illustrated in FIG. 2 includes a medical facility 5a, a medical facility 5b, the prediction device 1 communicably connected to each of the devices in the medical facility 5a and the 5b via the communication network 6, and the first prediction information management device 3 communicably connected to the prediction device 1.

The medical facility 5a includes a terminal device 2a, a subject image management device 4a, and an electronic medical record management device 9a, which are communicably connected to one another. On the other hand, the medical facility 5b includes a terminal device 2b, a subject image management device 4b, and an electronic medical record management device 9b, which are communicably connected to one another. In the description below, when the terminal devices 2a and 2b are not particularly distinguished from each other and the medical facilities 5a and 5b are not particularly distinguished from each other, terminal devices 2a and 2b and the medical facilities 5a and 5b are respectively referred to as the "terminal device 2" and the "medical facility 5".

FIG. 2 illustrates an example in which the LANs of the medical facilities 5a and 5b are connected to the communication network 6. The prediction device 1 is not limited to the configuration illustrated in FIG. 2, as long as the prediction device 1 is communicably connected to the devices in each medical facility via the communication network 6. For example, the prediction device 1 and the first prediction information management device 3 may be installed in the medical facility 5a or in the medical facility 5b.

The prediction system 100a having such a configuration may include the first prediction information management device 3a installed in the medical facility 5a and the first prediction information management device 3b installed in the medical facility 5b. In this case, the prediction device 1 can respectively acquire first prediction information and a subject image of a subject Pa from the first prediction information management device 3a and the subject image management device 4a of the medical facility 5a. The prediction device 1 transmits a prediction image obtained by predicting the condition of the target region of the subject Pa, to the terminal device 2a installed in the medical facility 5a. The prediction device 1 can respectively acquire first prediction information and a subject image of a subject Pb from the first prediction information management device 3b and the subject image management device 4b of the medical facility 5b. The prediction device 1 transmits a prediction image obtained by predicting the condition of the target region of the subject Pb, to the terminal device 2b installed in the medical facility 5a.

In this case, the first prediction information and the subject image of each subject may include identification information assigned to each medical facility 5 where the subject is examined, the identification information being unique to the medical facility 5, and identification information assigned to the subject and that is unique to the subject. The identification information unique to each medical facility 5 may be, for example, a facility ID. The identification information unique to each subject may be, for example, a patient ID. Based on these pieces of identification information, the prediction device 1 can correctly transmit the prediction image obtained by predicting the condition of the target region of the subject, to the terminal device 2 of each medical facility 5 where the subject is examined.

### Configurations of Prediction System 100 or 100a

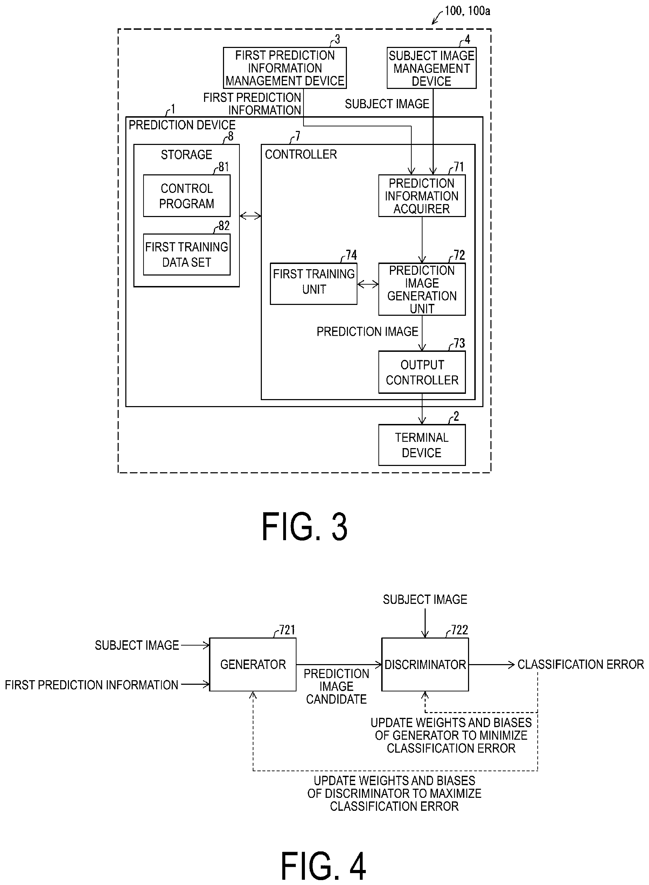

A configuration of a prediction system 100 or 100a will be described with reference to FIG. 3. FIG. 3 is a block diagram illustrating an example of the configuration of the prediction system 100 or 100a according to the aspect of the present disclosure. For convenience of description, members having the same functions as those already described are denoted by the same reference signs, and the description thereof is not repeated.

The prediction system 100 or 100a illustrated in FIG. 3 includes the prediction device 1, and one or more terminal devices 2, the first prediction information management device 3, and the subject image management device 4 communicably connected to the prediction device 1.

### Configuration of Prediction Device 1

The prediction device 1 includes a controller 7 that controls each unit of the prediction device 1 in an integrated manner, and a storage 8 that stores various data to be used by the controller 7. The controller 7 includes a prediction information acquirer 71, a prediction image generation unit 72, and an output controller 73. The storage 8 stores a control program 81, which is a program for executing various controls of the prediction device 1.

### Prediction Information Acquirer 71

The prediction information acquirer 71 acquires the subject image from the subject image management device 4 and acquires the first prediction information from the first prediction information management device 3. The subject image and the first prediction information are input data input to the prediction image generation unit 72.

The subject image and the first prediction information will be described by taking several disorders as examples. For example, when the disorder is obesity, the subject image may be an image of the whole body or abdomen of the current subject, and the first prediction information may be information regarding the weight, BMI, abdominal girth, visceral fat mass, blood pressure, blood glucose level, lipid, uric acid level or liver function level of the subject. For example, when the disorder is alopecia, the subject image may be an image representing the whole body or the head of the current subject, and the first prediction information may be information regarding the number of hairs, sex hormone level, Norwood scale, or Ludwig scale of the subject. For example, when the disorder is cataracts, the subject image may be an image representing the head (face) or eyes of the current subject, and the first prediction information may be information regarding the visual acuity, vision breadth, the level of clouding of the eye lens, or Emery-Little scale for the subject. For example, when the disorder is periodontal disease, the subject image may be an image representing the head (face) or oral cavity of the current subject, and the first prediction information may be information regarding the degree of pain in tooth or gingiva, the degree of swelling of tooth or gingiva, the number of remaining teeth, gingival index, and periodontal pocket depth for the subject. When the disorder is periodontal disease, the subject image may be an image representing an open mouth or an image representing a closed mouth. For example, when the disorder is rheumatoid arthritis, the subject image may be an image representing the current whole body, upper limb, or lower limb of the subject, and the first prediction information may be information regarding the degree of pain, the degree of swelling, joint angle, joint movable range, Larsen scale, or Stein blocker scale for the whole body, upper limb, or lower limb of the subject. For example, when the disorder is Heberden's node, the subject image may be an image representing the current hand of the subject, and the first prediction information may be information regarding the degree of pain, the degree of swelling, or joint movable range for the hand of the subject. For example, when the disorder is hallux valgus, the subject image may be an image representing the current foot of the subject, and the first prediction information may be information regarding the degree of pain, the degree of swelling, joint movable range, HV angle, or M1-M2 angle for the foot of the subject. For example, when the disorder is osteoarthritis, the subject image may be an image representing the current whole body, upper limb, or lower limb of the subject, and the first prediction information may be information regarding the degree of pain, the degree of swelling, joint angle, joint movable range, or K-L scale for the whole body, upper limb, or lower limb of the subject. For example, when the disorder is spondylosis deformans, the subject image may be an image representing the current whole body, neck, thorax, or waist of the subject, and the first prediction information may be information regarding the degree of spinal curvature, spinal motion range, or K-L scale for the subject. For example, when the disorder is compression fracture, the subject image may be an image representing the current whole body or waist of the subject, and the first prediction information may be information regarding the degree of spinal curvature, spinal motion range, or the K-L scale for the subject. For example, when the disorder is sarcopenia, the subject image may be an image representing the current whole body, upper limb, or lower limb of the subject, and the first prediction information may be information regarding the muscle mass of the subject. The subject image may be a medical image captured in medical examination of each disorder. For example, when the disorder is knee osteoarthritis, the subject image may be an X-ray image representing the knee joint of the current subject, and the first prediction information may be information regarding the angle between the tibia and the femur of the subject two years later.

### Prediction Image Generation Unit 72

The prediction image generation unit 72 generates a prediction image from the subject image by predicting the condition of the target region at the second time based on the first prediction information, and outputs the prediction image. The prediction image generation unit 72 may generate an image that imitates at least a part of the subject image used to generate the prediction image. The prediction image generated by the prediction image generation unit 72 may be an image indicating the effect, on the target region, of a disorder occurring in the target region. The generated prediction image may include an image associated with a region of the subject that remains unchanged at the second time with respect to the first time. That is, the prediction image may include an image associated with a region that has changed between the first time and the second time and an image associated with a region that remains unchanged between the first time and the second time.

The prediction image generation unit 72 may have any known image editing function and moving image editing function. In this case, the prediction image generation unit 72 converts the subject image into an editable file format, and then makes, in the subject image, a change based on the first prediction information, to generate the prediction image. For example, when the subject image is an image of the lower limb of the current subject and the first prediction information is information regarding the angle formed by the tibia and the femur of the subject two years later, the prediction image generation unit 72 first converts the subject image into a predetermined file format. The prediction image generation unit 72 may generate a prediction image by changing, based on the first prediction information, the angle formed by the tibia and the femur appearing in the subject image whose file format has been converted.

The prediction image generation unit 72 may include a prediction image generation model that can generate a prediction image using the subject image and the first prediction information. Here, the prediction image generation model may be a neural network trained using, as teacher data, a plurality pieces of image data each representing the target region. For example, a convolutional neural network (CNN), a generative adversarial network (GAN), or an auto encoder may be applied as the prediction image generation model.

The prediction image generation unit 72 inputs the subject image and the first prediction information to the prediction image generation model and causes the prediction image generation model to output a prediction image. The prediction image generation unit 72 outputs the prediction image output from the prediction image generation model (that is, generated by the prediction image generation unit 72).

The prediction image generation model is a calculation model used by the prediction image generation unit 72 when executing calculations based on the input data. The prediction image generation model is generated by executing machine learning, which will be described below, on the neural network included in the prediction image generation unit 72.

### Output Controller 73

The output controller 73 transmits, to the terminal device 2, the prediction image output from the prediction image generation unit 72. The output controller 73 may transmit, to the terminal device 2 together with the prediction image, the subject image and/or the first prediction information used to generate the prediction image.

The prediction device 1 may include a display (not illustrated). In that case, the output controller 73 may cause the display to display the prediction image. In this case, the output controller 73 may cause the display unit to display, together with the prediction image, the subject image and/or the first prediction information used to generate the prediction image.

By including the prediction image generation unit 72 including the prediction image generation model, the prediction system 100 or 100a can generate and output a realistic prediction image obtained by reflecting the condition of the target region at the second time in the subject image. Accordingly, the prediction system 100 or 100a can make the subject clearly recognize the condition of the target region of the subject at the second time.

In one example, a trained prediction image generation model may be installed in the prediction device 1 in advance. Alternatively, the prediction device 1 may further include a first training unit 74 that executes training processing on the prediction image generation unit 72.

### First Training Unit 74

The first training unit 74 controls training processing on the neural network included in the prediction image generation unit 72.

### Training Processing of Prediction Image Generation Model

Hereinafter, with reference to FIG. 4, training processing will be described that is intended to generate a prediction image generation model to which a generative adversarial network (GAN) is applied. FIG. 4 is a diagram illustrating an example of a configuration of a neural network included in the prediction image generation unit 72.

As illustrated in FIG. 4, the prediction image generation model to which the generative adversarial network is applied includes two networks, a generator network (hereinafter referred to as a generator 721) and a discriminator network (hereinafter referred to as a discriminator 722). The generator 721 can generate an image that appears to be a real image, from the first prediction information and the subject image as a prediction image. On the other hand, the discriminator 722 can discriminate between the image data (false image) from the generator 721 and a real image from a first training data set 82 described below.

First, the first training unit 74 acquires the subject image and the first prediction information from the storage 8 and inputs the subject image and the first prediction information to the generator 721.

The generator 721 generates a prediction image candidate (false image) from the subject image and the first prediction information. The generator 721 may generate the prediction image candidate with reference to the real image included in the first training data set 82.

The first training data set 82 is data used for machine learning for generating the prediction image generation model. The first training data set 82 may include any real images used as targets to be reproduced as faithfully as possible by the generator 721. For example, the first training data set 82 may include real medical images captured in the past. The medical image may include, for example, at least one selected from the group consisting of X-ray image data, CT image data, MRI image data, PET image data, and ultrasonic image data representing a captured image of the target region of each of a plurality of patients.

The first training data set 82 may include first training data and first teacher data. The first training data includes, for example, data of a type same as and/or similar to the subject image and data of a type same as and/or similar to the first prediction information. The "data of a type same as and/or similar to the subject image" means image data obtained by capturing, at the same angle as that for the subject image, an image of the same target region as that appearing in the subject image, and image data of the same image type as the image, such as a medical image or an appearance image. The "data of a type same as and/or similar to the first prediction information" means, when the first prediction information is information related to the shape and appearance of a target region associated with a disorder, information related to the shape and appearance of the same target region associated with the same disorder. The first teacher data is data of a type same as and/or similar to the prediction image and is data of the same person for which time has elapsed with respect to the first training data. The first teacher data is data regarding "data of a type same as and/or similar to the first prediction information" which is the first training data. The "data of a type same as and/or similar to the prediction image" is image data obtained by capturing an image of the same target region as that appearing in the prediction image, at the same angle as that for the prediction image, and means image data of the same image type such as a medical image or an appearance image.

Then, the first training unit 74 inputs, to the discriminator 722, prediction image candidates generated by the generator 721 and the real image included in the first training data set 82.

The discriminator 722 takes, as inputs, the real image from the first training data set 82 and the prediction image candidates generated by the generator 721, and outputs, for each image, a probability that the image is real.

The first training unit 74 calculates a classification error indicating how correct the probability output by the discriminator 722 is. The first training unit 74 iteratively improves the discriminator 722 and the generator 721 using the error back-propagation method. At this time, weights and biases of the discriminator 722 are updated to minimize the classification error (that is, to maximize discrimination performance). On the other hand, the weights and biases of the generator 721 are updated to maximize the classification error (that is, to maximize the probability that the discriminator 722 mistakes the prediction image candidate for the real image).

The first training unit 74 updates the weights and the biases of the discriminator 722 and the weights and the biases of the generator 721 until the probability output by the discriminator 722 satisfies a predetermined criterion. Thus, the prediction image generation unit 72 can generate a prediction image that is indistinguishable from the real image.

### Processing Executed by Prediction System 100 or 100a

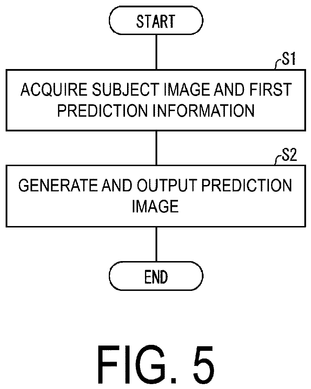

The flow of processing executed by the prediction system 100 or 100a will be described below with reference to FIG. 5. FIG. 5 is a flowchart illustrating an example of flow of processing executed by the prediction system 100 or 100a according to the present embodiment.

First, in step S1, the prediction information acquirer 71 acquires a subject image and first prediction information (input data) (prediction information acquiring step).

Subsequently, in response to the input of the subject image and the first prediction information, the prediction image generation unit 72 generates a prediction image and outputs the prediction image in step S2 (prediction image generation step).

### Second Embodiment

Another embodiment of the present disclosure will be described below. For convenience of description, a member having the same function as that of a member described in the embodiments described above is denoted by the same reference sign, and description thereof will not be repeated.

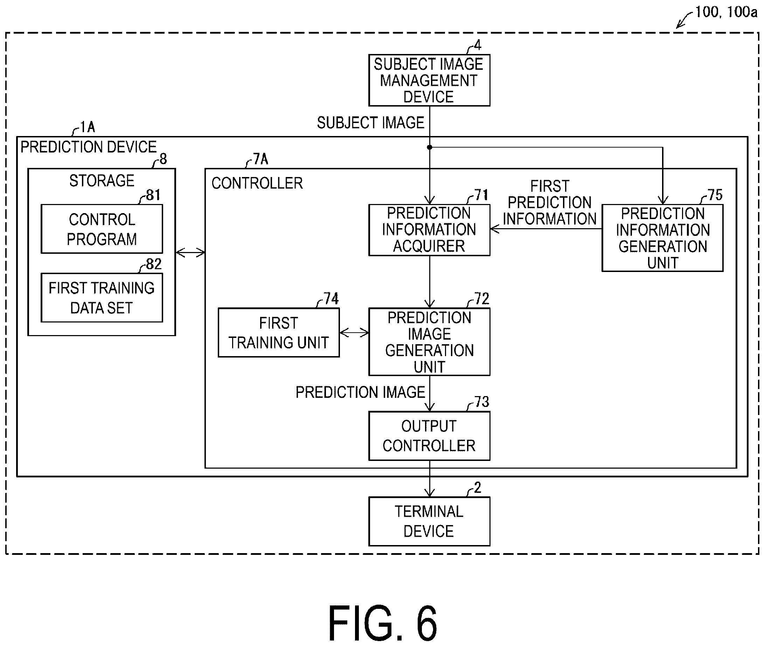

The prediction system 100 or 100a according to the above-described embodiment includes the prediction device 1 that acquires the first prediction information from the first prediction information management device 3 but is not limited to this configuration. For example, a prediction device 1A may generate the first prediction information. The configuration of the prediction system 100 or 100a including the prediction device 1A will be described with reference to FIG. 6. FIG. 6 is a block diagram illustrating an example of configuration of the prediction system 100 or 100a according to another aspect of the present disclosure.

### Configuration of Prediction Device 1A

The prediction device 1A includes a controller 7A that controls each unit of the prediction device 1A in an integrated manner, and the storage 8 that stores various data to be used by the controller 7A. The controller 7A further includes a prediction information generation unit 75 in addition to the prediction information acquirer 71, the prediction image generation unit 72, and the output controller 73.

FIG. 6 illustrates an example in which the prediction device 1A includes the first training unit 74, but the present disclosure is not limited to this configuration. In one example, a trained prediction image generation model may be installed in the prediction device 1A in advance.

### Prediction Information Generation Unit 75

The prediction information generation unit 75 generates, from a subject image representing a target region of a subject at a first time, first prediction information regarding the target region at a second time when a predetermined period has elapsed from the first time, and outputs the first prediction information to the prediction information acquirer 71.

The prediction information generation unit 75 may include a prediction information generation model that can estimate the first prediction information from the subject image. The prediction information generation model is a model that can estimate the first prediction information from the subject image of the subject and the basic information of the subject. Here, the prediction information generation model may be a neural network trained by using, as teacher data, patient information regarding a patient having a disorder of the target region. For example, a convolutional neural network (CNN), a recurrent neural network (RNN), and a Long Short-Term Memory (LSTM) may be used as the prediction information generation model.

The patient information is, for example, information that includes condition information indicating the condition of the target region of each patient acquired at a plurality of past times and in which the condition information for each patient is associated with information indicating the time at which the condition information is acquired.

The prediction information generation unit 75 inputs data regarding the subject image to the prediction information generation model and causes the prediction information generation model to output the first prediction information. The prediction information generation unit 75 outputs the first prediction information output from the prediction information generation model (that is, generated by the prediction information generation unit 75).

The prediction information generation model is a calculation model used by the prediction information generation unit 75 when executing calculations based on the input data. The prediction information generation model is generated by executing machine learning, which will be described below, on the neural network included in the prediction information generation unit 75.

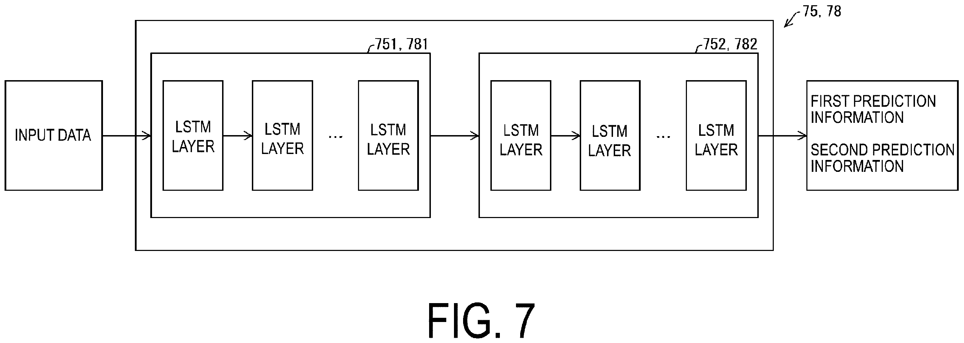

Hereinafter, the configuration of the prediction information generation unit 75 will be further described with reference to FIG. 7 by taking, as an example, application of the neural network as a prediction information generation model. FIG. 7 is a diagram illustrating an example of a configuration of the neural network included in the prediction information generation unit.

As illustrated in FIG. 7, the prediction information generation unit 75 includes an input layer 751 and an output layer 752. The prediction information generation unit 75 executes calculations based on the prediction information generation model on the input data input to the input layer 751, and outputs prediction information from the output layer 752.

In FIG. 7, the prediction information generation unit 75 includes a neural network including the input layer 751 and the output layer 752. The neural network is a neural network suitable for handling time series information. The neural network may be, for example, an LSTM. The neural network may be a neural network suitable for handling time series information and position information in combination. The neural network is, for example, a ConvLSTM network, which is a combination of CNN and LSTM. The input layer 751 can extract a feature of a temporal change in the input data. The output layer 752 can calculate a new feature based on the feature extracted by the input layer 751 and the temporal change in and the initial value of the input data. Each of the input layer 751 and the output layer 752 includes a plurality of LSTM layers. Each of the input layer 751 and the output layer 752 may include three or more LSTM layers.

The input data input to the input layer 751 may be, for example, a parameter indicating a feature extracted from a subject image representing the target region of the subject at the first time. In this case, the prediction information generation unit 75 can output the first prediction information regarding the target region at the second time when the predetermined period has elapsed from the first time.

The prediction information generation unit 75 outputs, as the first prediction information, for example, prediction results for the onset of or the degree of progression of a disorder related to the target region of the subject at the second time when the predetermined period has elapsed from the first time. Specifically, the prediction information generation unit 75 outputs, as the first prediction information, for example, the degree of the symptom of each disorder of the subject at the second time, the classification in each disorder, and information indicating the time when invasive treatment is required for the target region. The first prediction information indicated here is an example and is not limited thereto.

Based on the first prediction information described above, the prediction information generation unit 75 may output information indicating the QOL of the subject as third prediction information. Specifically, the prediction information generation unit 75 outputs, as the third prediction information, at least one of information regarding the pain developing in the target region of the subject, information regarding the catastrophizing of the subject, information regarding the motor ability of the subject, information indicating the level of life satisfaction of the subject, and information regarding the level of stiffness of target region of the subject.

The information indicating the QOL of the subject is information including at least one of the following.
- Information regarding the pain developing in the target region of the subject
- Information regarding catastrophizing of the subject
- Information regarding the motor ability of the subject
- Information indicating the level of life satisfaction of the subject

The information indicating the QOL of the subject may include the subject's (1) physical functionality, (2) physical role functionality, (3) bodily pain, (4) general health perceptions, (5) vitality, (6) social role functionality, (7) emotional roll functionality, and (8) mental health.

The information regarding QOL may include, for example, SF-36 (36-Item. Short-Form Health Survey), VAS (Visual analog scale), NEI VFQ-25 (The 25-item National Eye Institute Visual Function Questionnaire), GOHAI (General Oral Health Assessment Index), WOMAC (Western Ontario and McMaster Universities Osteoarthritis Index), RDQ (Roland-Morris Disability Questionnaire) and other information.

The prediction information generation unit 75 may generate at least a part of the first prediction information used by the prediction image generation unit 72 to generate the prediction image. In this case, the remaining first prediction information may be acquired by the prediction information acquirer 71 from the first prediction information management device 3.

### Processing Executed by Prediction System 100 or 100a

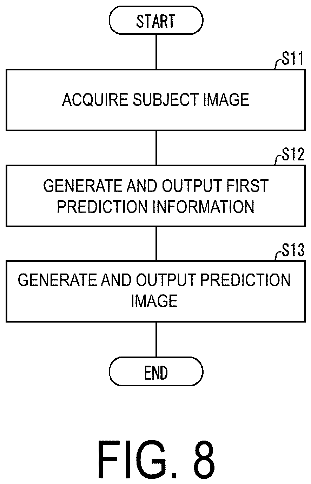

The flow of processing executed by the prediction system 100 or 100a according to the second embodiment will be described below with reference to FIG. 8. FIG. 8 is a flowchart illustrating an example of the flow of processing executed by the prediction system 100 or 100a according to the second embodiment.

First, in step S11, the prediction information generation unit 75 acquires a subject image (input data) (image acquiring step).

Subsequently, in response to the input of the subject image, in step S12, the prediction information generation unit 75 generates the first prediction information and outputs the first prediction information to the prediction information acquirer 71 (first prediction step).

Then, the prediction information acquirer 71 inputs, to the prediction image generation unit 72 (not illustrated), (a) the first prediction information acquired from the prediction information generation unit 75 and (b) the subject image (input data) acquired from the subject image management device 4 before the acquisition of the first prediction information or at the same time as or after the acquisition of the first prediction information.

Subsequently, in response to the input of the subject image and the first prediction information, the prediction image generation unit 72 generates a prediction image and outputs the prediction image in step S13 (prediction image generation step).

### Variation

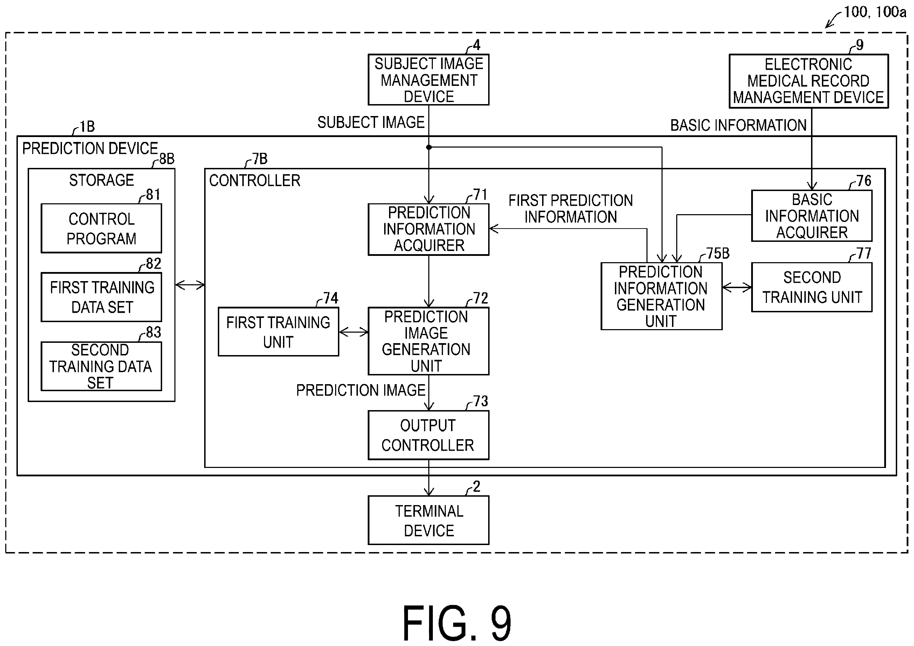

In the prediction device 1A, the prediction information generation unit 75 generates the first prediction information based on the subject image. On the other hand, in a prediction device 1B according to the present variation of the second embodiment, a prediction information generation unit 75B generates first prediction information based on the basic information in addition to the subject image. The configuration of the prediction system 100 or 100a including the prediction device 1B will be described with reference to FIG. 9. FIG. 9 is a block diagram illustrating a variation of configuration of the prediction system 100 or 100a according to another aspect of the present disclosure. The prediction device 1B further includes the prediction information generation unit 75B in a controller 7B.

The prediction device 1 includes the controller 7B that controls each unit of the prediction device 1 in an integrated manner, and a storage 8B that stores various data to be used by the controller 7B. The controller 7B further includes the prediction information generation unit 75B and a basic information acquirer 76 in addition to the prediction information acquirer 71, the prediction image generation unit 72, and the output controller 73.

With the above-described configuration, the prediction device 1B can generate information regarding a symptom closer to a symptom that may occur or has occurred in the target region at the second time, that is, more accurate first prediction information, based on the subject image captured at the first time. As a result, the prediction device 1B can generate an image indicating a symptom closer to a symptom that may occur or has occurred in the target region at the second time, that is, a prediction image indicating more accurate prediction information.

### Basic Information Acquirer 76

The basic information acquirer 76 acquires basic information, which is information regarding the subject, from the electronic medical record management device 9. The electronic medical record management device 9 is a computer that functions as a server for managing electronic medical record information of a subject who is examined in the medical facility 5 or in a medical facility other than the medical facility 5. The electronic medical record information may include basic information and medical interview information about the subject. The basic information is input data to be input to the prediction information generation unit 75B in addition to the subject image.

The basic information is information including at least one selected from the group consisting of the sex, age, height, weight of the subject, and information indicating the condition of the target region of the subject at the first time. The basic information may further include at least one of the body mass index (BMI), race, occupational history, exercise history, history of target region disorder, information regarding the target region shape and appearance, biomarker information, and genetic information of the subject. The basic information may include, for example, information such as the degree of the symptom of the disorder related to the target region of the subject. The basic information may include, for example, information included in the electronic medical record information of the subject. The basic information may be medical interview information acquired from the subject through a medical interview conducted in the medical facility 5 or the like and may include, for example, information regarding the QOL of the subject at the first time.

According to the configuration described above, the prediction device 1B acquires the basic information of the subject from the electronic medical record management device 9 in addition to the subject image, and transmits the prediction image obtained by predicting the target region of the subject Pa, to the terminal device 2a installed in the medical facility 5a. According to the configuration described above, the prediction device 1B acquires the basic information of the subject from the electronic medical record management device 9 in addition to the subject image, and transmits the prediction image obtained by predicting the target region of the subject Pb, to the terminal device 2b installed in the medical facility 5b.

In one example, the trained prediction information generation model may be installed in the prediction device 1B in advance. Alternatively, the prediction device 1B may include a second training unit 77 that executes training processing on the prediction information generation unit 75B.

### Second Training Unit 77

The second training unit 77 controls training processing on the neural network included in the prediction information generation unit 75B. This training includes the use of a second training data set 83 described below. A specific example of training executed by the second training unit 77 will be described below.

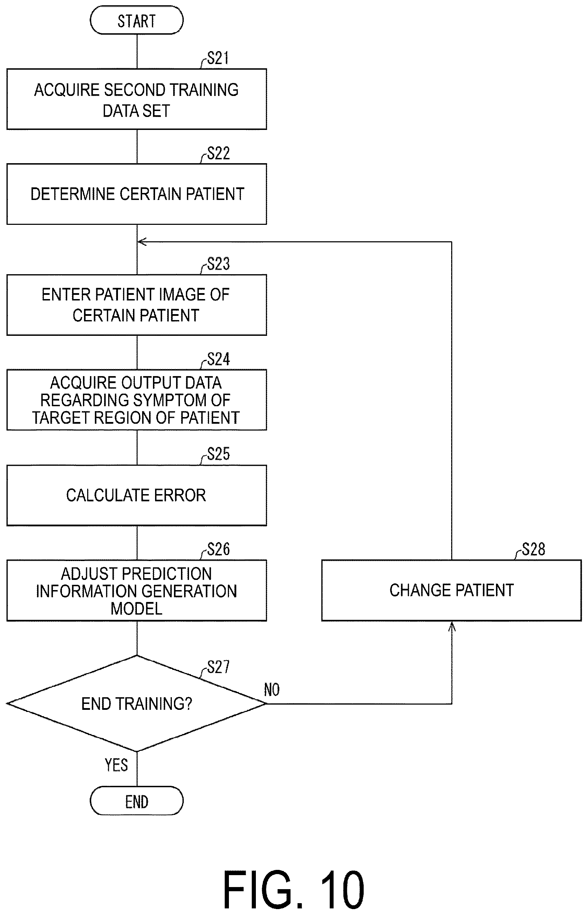

Training Processing of Prediction Information Generation Model Hereinafter, with reference to FIG. 10, training processing will be described that is intended to generate a prediction information generation model to which a neural network is applied. FIG. 10 is a flowchart illustrating an example of a flow of training processing on the neural network included in the prediction information generation unit 75B.

First, the second training unit 77 acquires, from the storage 8B, second training data included in the second training data set 83 (step S21). The second training data includes patient images of a plurality of patients.

Subsequently, the second training unit 77 determines a certain patient (step S22).

Subsequently, the second training unit 77 inputs, to the input layer 751, a patient image of the certain patient at a time A, which is included in the second training data (step S23). The input layer 751 may extract a parameter indicating a feature from the input patient image.

Then, the second training unit 77 acquires output data regarding the symptom of the target region of the certain patient from the output layer 752 (step S24). The output data has the same contents as those of the second teacher data.

Subsequently, the second training unit 77 acquires the second teacher data included in the second training data set 83. Then, the second training unit 77 compares the acquired output data with condition information indicating the condition of the target region of the certain patient at a time B and included in the second teacher data. The second training unit 77 then calculates errors (step S25).

Then, the second training unit 77 adjusts the prediction information generation model to reduce the errors (step S26).

Any known method is applicable to adjustment of the prediction information generation model. For example, the error back-propagation method may be employed as a method for adjusting the prediction information generation model. The adjusted prediction information generation model is a new prediction information generation model, and the prediction information generation unit 75B uses the new prediction information generation model in the subsequent calculation. In the adjustment stage of the prediction information generation model, parameters used in the prediction information generation unit 75B may be adjusted.

The parameters include, for example, parameters used in the input layer 751 and the output layer 752. Specifically, the parameters include weighting factors used in the input layer 751 and in the LSTM layers of the output layer 752. The parameters may include a filter coefficient.

When the error is not within a predetermined range, and the patient images of all the patients included in the second training data set 83 are not input (NO in step S27), the second training unit 77 changes the patient (step S28), further returns to step S23, and repeats the training processing. When the error is within the predetermined range, and the patient images of all the patients included in the second training data set 83 are input (YES in step S27), the second training unit 77 ends the training processing.

### Second Training Data Set 83

The second training data set 83 is data used for machine learning for generating a prediction information generation model. The second training data set 83 may include patient information regarding a patient having a disorder of the target region. Here, the patient information may include condition information indicating the condition of the target region of each patient and acquired at a plurality of past times and may be information in which the condition information for each patient is associated with information indicating the time at which the condition information is acquired. The second training data set 83 includes second training data used as input data and second teacher data for calculating a difference from the first prediction information output by the prediction information generation unit 75B.

For example, the second training data may include image data representing the target region of each of the plurality of patients. The image data used as the second training data may be captured image data representing a captured image of any one of the whole body, upper body, lower body, upper limb, and lower limb of each of the plurality of patients. The image data used as the second training data may be medical image data representing the target region of each of the plurality of patients. The medical image data may include, for example, at least one selected from the group consisting of X-ray image data, CT image data, MRI image data, PET image data, and ultrasonic image data representing a captured image of the target region of each of the plurality of subjects. The second training data is the data of a type same as and/or similar to the subject image. The second teacher data is the data of a type same as and/or similar to the first prediction information.

The second teacher data may include condition information indicating the condition of the target region of each patient at the time when the patient image is captured, and symptom information regarding the target region. The condition information may include information regarding the progression of disorder of the target region. The symptom information may include information regarding the onset timing of disorder of the target region.

The second training data set 83 may include the second training data and the second teacher data integrated together. In other words, the second training data set 83 may be time series data in which patient images acquired from each of the plurality of patients at a plurality of times in the past are associated with condition information indicating the condition of the target region at the time when the patient image is captured. For example, the second training data set 83 may include parameters indicating features extracted from the following information at a certain time and one year after the certain time.

For example, when the disorder is obesity, the second training data set 83 may include weight, BMI, abdominal girth, visceral fat mass, blood pressure, blood glucose level, lipid, uric acid level, or liver function level. For example, when the disorder is alopecia, the second training data set 83 may include the number of hairs, sex hormone level, Norwood scale, or Ludwig scale. For example, when the disorder is cataracts, the second training data set 83 may include visual acuity, vision breadth, the level of clouding of the eye recommendation, or Emery-Little scale. For example, when the disorder is periodontal disease, the second training data set 83 may include a degree of pain in teeth or gums, a degree of swelling in teeth or gums, the number of remaining teeth, a gingival index, or a periodontal pocket depth. For example, when the disorder is rheumatoid arthritis, the second training data set 83 may include the degree of pain, the degree of swelling, the joint angle, the joint movable range, the Larsen scale, or the Stein blocker scale for the whole body, the upper limb, or the lower limb of the subject. For example, when the disorder is Heberden's node, the second training data set 83 may include the degree of pain, the degree of swelling, or joint movable range for the hand of the subject. For example, when the disorder is hallux valgus, the second training data set 83 may include the degree of pain, the degree of swelling, joint movable range, HV angle, or M1-M2 angle for the feet for the subject. For example, when the disorder is osteoarthritis, the second training data set 83 may include the degree of pain, the degree of swelling, joint angle, joint movable range, the level of stiffness, joint cartilage thickness, K-L scale, or the presence of claudication for the whole body, the upper limb, or the lower limb of the subject. For example, when the disorder is spondylosis deformans, the second training data set 83 may include the degree of pain, the degree of spinal curvature, spinal motion range, or the K-L scale. For example, when the disorder is compression fracture, the second training data set 83 may include the degree of pain or spinal motion range. For example, when the disorder is sarcopenia, muscle mass, walking speed, or grip strength may be included.

The second training data set 83 may also include parameters indicating attributes of the subject. The attributes of the subject are, for example, the sex, age, height, and weight of each subject. When the second training data set 83 is time series data, the second training unit 77 may use a subject image at a certain time as the second training data, and may use, as the second teacher data, a subject image when a predetermined period has elapsed from the certain time, information regarding the symptom of the target region at the time when the subject image is taken, and information regarding the subject.

The second training data set 83 may include information regarding the QOL of each of the plurality of subjects in the time series data. For example, information of SF-36, or VAS may be included. The prediction information generation unit 75B including the prediction information generation model generated by machine learning using the second training data set 83 can output, from the subject image of the subject, information related to the QOL of the subject at the second time.

Specifically, the input data used during training of the prediction information generation unit 75B is (a) a subject image representing the target region of the subject at a certain time A that is included in the second training data. Based on the input data described above, the prediction information generation unit 75B outputs, as the output data, first prediction information regarding the target region at a time B when a predetermined period (for example, three years) has elapsed from the time A. Specifically, for example, the prediction information generation unit 75B outputs, as the output data, for example, information indicating the angle around the target region of the subject at the time B, the degree of enlargement and shrinkage of the target region, the degree of wrinkles and spots on the target region, the time when pain develops in the target region and the degree of the pain, and the time when invasive treatment is required for the target region. The output data indicated here are examples and are not limited thereto.

When the prediction information generation unit 75B uses the basic information as input data, the second training unit 77 may input, when training the prediction information generation unit 75B, the symptom information and attribute information of the general subject to the prediction information generation unit 75B as the second training data, in addition to the subject image representing the target region of the subject at the certain time A.

### Processing Executed by Prediction System 100 or 100

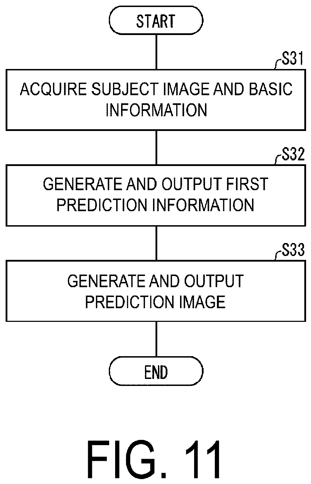

The flow of processing executed by the prediction system 100 or 100a including the prediction device 1B will be described below with reference to FIG. 11. FIG. 11 is a flowchart illustrating the flow of processing executed by the prediction system 100 or 100a according to the present embodiment.

First, in step S31, the prediction information generation unit 75B acquires a subject image (input data) and basic information (input data) (image and information acquiring step).

Subsequently, in response to the input of the subject image and the basic information, the prediction information generation unit 75B generates first prediction information in step S32 and outputs the first prediction information to the prediction information acquirer 71 (first prediction step).

Then, the prediction information acquirer 71 inputs, to the prediction image generation unit 72, (a) the first prediction information acquired from the prediction information generation unit 75B and (b) the subject image (input data) acquired from the subject image management device 4 before the acquisition of the first prediction information or simultaneously with or after the acquisition of the first prediction information (not illustrated).

Subsequently, in response to the input of the subject image and the first prediction information, the prediction image generation unit 72 generates a prediction image and outputs the prediction image in step S33 (prediction image generation step).

### Third Embodiment

Another embodiment of the present disclosure will be described below. For convenience of description, a member having the same function as that of a member described in the embodiments described above is denoted by the same reference sign, and description thereof will not be repeated.

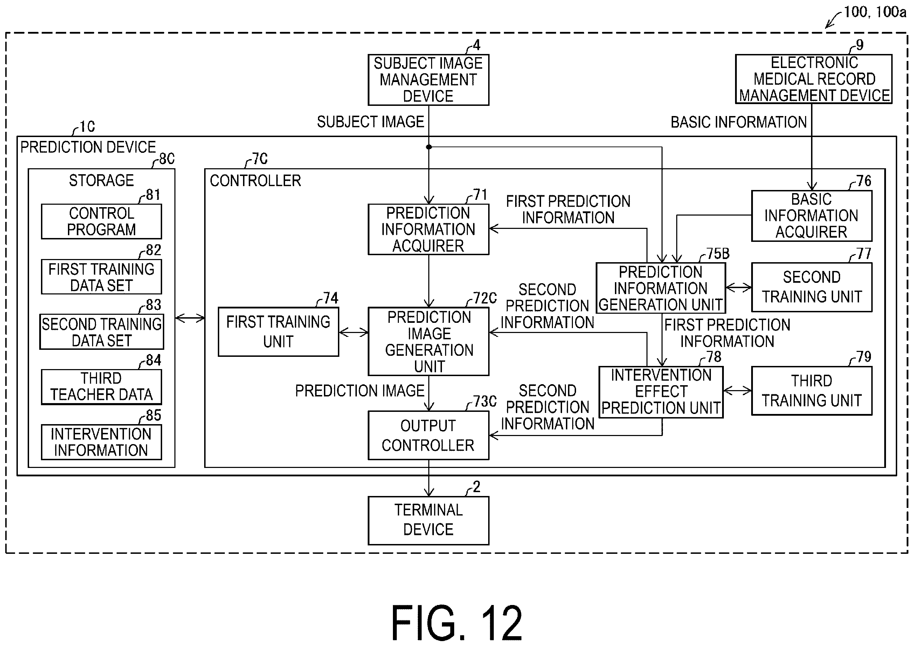

The prediction system 100 or 100a may have a function to output a prediction image obtained by predicting the condition of the target region at the second time when intervention in the target region is present, a method for intervention in the subject, and the effect of the intervention. The prediction device 1C including such a function will be described with reference to FIG. 12. FIG. 12 is a block diagram illustrating an example of configuration of the prediction system 100 or 100a according to another aspect of the present disclosure.

Here, a specific example of the method for intervention will be described. For example, when the disorder is obesity, the method for intervention may include lifestyle guidance, dietetic therapy, drug therapy, exercise therapy, and surgical therapy (liposuction, gastrectomy, and gastric banding). For example, when the disorder is alopecia, the method for intervention may include lifestyle guidance, dietetic therapy, drug therapy, surgical therapy (hair transplant surgery), and wig wear. For example, when the disorder is cataracts, the method for intervention may include drug therapy, exercise therapy, and surgical therapy (cataract extraction and intraocular lens placement). For example, when the disorder is periodontal disease, the method for intervention may include oral care instructions, drug therapy, corrective therapy, surgical therapy (periodontal plastic surgery and implant treatment), and use of dentures. For example, when the disorder is rheumatoid arthritis, the method for intervention may include drug therapy and surgical therapy (osteotomy and joint replacement). For example, when the disorder is Heberden's node, the method for intervention may include drug therapy. For example, when the disorder is hallux valgus, the method for intervention may include shoe instructions, exercise therapy, orthotic therapy, drug therapy, and surgical therapy (osteotomy, fusion, and joint replacement). For example, when the disorder is osteoarthritis, the method for intervention may include exercise therapy, orthotic therapy, drug therapy, rehabilitation, and surgical therapy (intra-articular injection, arthroscopic surgery, osteotomy, fusion, and joint replacement). For example, when the disorder is spondylosis deformans, the method for intervention may include exercise therapy, orthotic therapy, drug therapy, and surgical therapy (spinal instrumentation surgery). For example, when the disorder is compression fracture, the method for intervention may include orthotic therapy, drug therapy, and surgical therapy (spinal instrumentation surgery). For example, when the disorder is sarcopenia, the method for intervention may include lifestyle guidance, dietetic therapy, drug therapy, and exercise therapy.

### Configuration of Prediction Device 1

The prediction device 1 includes a controller 7C that controls each unit of the prediction device 1 in an integrated manner, and a storage 8C that stores various data to be used by the controller 7C. The controller 7C includes an intervention effect prediction unit 78 and a third training unit 79, in addition to a prediction information acquirer 71, a prediction image generation unit 72C, an output controller 73C, the first training unit 74, the prediction information generation unit 75B, the basic information acquirer 76, and the second training unit 77.

Although FIG. 12 illustrates the prediction device 1C including the first training unit 74, the second training unit 77, and the third training unit 79, the present disclosure is not limited to this configuration. The prediction device 1C may include any (or all) of the first training unit 74, the second training unit 77, and the third training unit 79 or may exclude any (or all) of these units.

For example, the prediction device 1C may exclude the first training unit 74. In this case, the trained prediction image generation model may be installed in the prediction device 1C in advance. Alternatively, the prediction device 1C may exclude the second training unit 77. In this case, the trained prediction information generation model may be installed in the prediction device 1C in advance. Alternatively, the prediction device 1C may exclude the third training unit 79. In this case, a trained intervention effect prediction model (described below) may be installed in the prediction device 1C in advance.

The storage 8C may store third teacher data 84 and intervention information 85 described below, in addition to the control program 81, which is a program for executing various controls of the prediction device 1, the first training data set 82, and the second training data set 83.

In the prediction device 1C illustrated in FIG. 12, the storage 8C stores the control program 81, the first training data set 82, the second training data set 83, the third teacher data 84, and the intervention information 85, but the present disclosure is not limited to this configuration. The storage 8C of the prediction device 1C may store any (or all) of the control program 81, the first training data set 82, the second training data set 83, the third teacher data 84, and the intervention information 85, or may avoid storing any (or all) of the program, data, and information.

### Prediction Image Generation Unit 72C

The prediction image generation unit 72C inputs a subject image, first prediction information, and second prediction information to the prediction image generation model and causes the prediction image generation model to output a prediction image. The prediction image generation unit 72C outputs the prediction image output from the prediction image generation model (that is, generated by the prediction image generation unit 72C).

### Output Controller 73C

The output controller 73C transmits, to the terminal device 2, the prediction image output from the prediction image generation unit 72C. As illustrated in FIG. 12, the output controller 73C may transmit, to the terminal device 2 together with the prediction image, at least one selected from the group consisting of the subject image, the first prediction information, and the second prediction information used to generate the prediction image.

### Intervention Effect Prediction Unit 78

The intervention effect prediction unit 78 outputs second prediction information indicating the method for intervention in the subject and the effect of the intervention, from the first prediction information regarding the target region at the second time when the predetermined period has elapsed from the first time. The intervention effect prediction unit 78 may include an intervention effect prediction model that can estimate the second prediction information from the first prediction information.

The intervention effect prediction model is a calculation model used by the intervention effect prediction unit 78 when executing calculations based on the input data. As long as the intervention effect prediction model is a calculation model that can estimate the second prediction information from the first prediction information, other configurations are not particularly limited.

The intervention effect prediction model may be a neural network, for example, a trained neural network including an input layer and an output layer. More specifically, the intervention effect prediction model may be a neural network trained by using effect information as teacher data.

For example, the effect information includes condition information indicating the condition of the target region of each patient which information is acquired at a plurality of times in the past, and is information in which the condition information for each patient is associated with the intervention information 85 indicating the intervention applied to each patient. The effect information may include time series data regarding the condition information of each of a plurality of patients to whom the intervention is applied in the past, the condition information having been acquired from each patient at a plurality of times in the past.

When the intervention effect prediction model is a trained neural network, in response to the first prediction information being input to the input layer as input data, the intervention effect prediction unit 78 performs calculation based on the intervention effect prediction model and outputs the second prediction information from the output layer as output data.

The second prediction information is, for example, information indicating the type of intervention and the effect of the intervention. The effect of the intervention is information representing the symptom of the target region of the subject at the second time when the intervention is applied. The effect of the intervention may be information representing the degree of improvement in the symptom of the disorder or the degree of suppression of progression of the symptom, for the target region of the subject at the second time as a result of application of the intervention as compared with a case where the intervention is not applied. The second prediction information may include information indicating the timing when the intervention is applied (intervention timing).

As an example, the intervention effect prediction unit 78 may be configured to extract a feature from the first prediction information and use the feature as input data. Known algorithms, such as those listed below, can be applied to extract the feature.
- Convolutional neural network (CNN)
- Auto encoder
- Recurrent neural network (RNN)
- Long short-term memory (LSTM)

Hereinafter, the configuration of the intervention effect prediction unit 78 will be further described with reference to FIG. 7 by taking, as an example, a case where the intervention effect prediction unit 78 is a neural network as an intervention effect prediction model. The configuration illustrated in FIG. 7 is merely an example, and the configuration of the intervention effect prediction unit 78 is not limited to this.

As illustrated in FIG. 7, the intervention effect prediction unit 78 includes an input layer 781 and an output layer 782. The intervention effect prediction unit 78 acquires the first prediction information from the prediction information generation unit 75B and uses the acquired first prediction information as input data to be input to the input layer 781. The intervention effect prediction unit 78 may further acquire a subject image and use the acquired subject image as input data. The intervention effect prediction unit 78 may acquire the basic information from the basic information acquirer 76 and use the acquired basic information as input data to be input to the input layer 781. The intervention effect prediction unit 78 executes calculations based on the intervention effect prediction model, on the input data input to the input layer 781, and outputs the prediction image from the output layer 782.

As illustrated in FIG. 7, the intervention effect prediction unit 78 includes a neural network including the input layer 781 and the output layer 782. The neural network is a neural network suitable for handling time series information. The neural network may be, for example, an LSTM. The neural network may be a neural network suitable for handling time series information and position information in combination. The neural network is, for example, a ConvLSTM network, which is a combination of CNN and LSTM. The input layer 781 can extract a feature of a temporal change in the input data. The output layer 782 can calculate a new feature based on the feature extracted in the input layer 781 and the temporal change in and the initial value of the input data. Each of the input layer 781 and the output layer 782 includes a plurality of LSTM layers. Each of the input layer 781 and the output layer 782 may include three or more LSTM layers.

The intervention effect prediction model is generated by executing machine learning, which will be described below, on the neural network included in the intervention effect prediction unit 78.

The input data input to the input layer 781 may be, for example, a parameter indicating a feature extracted from the first prediction information regarding the target region at the second time when the predetermined period has elapsed from the first time. Alternatively, the input data may be information indicating the method for intervention and included in the intervention information 85 described below. When using the intervention information 85 as input data, the intervention effect prediction unit 78 may select at least one of the methods for intervention included in the intervention information 85 and output second prediction information obtained by predicting the effect of the intervention.

In response to input of the above-described input data to the input layer 781, the output layer 782 outputs second prediction information indicating the method for intervention in the subject and the effect of the intervention.

The second prediction information may represent, for example, the degree to which the symptom of the disorder related to the target region of the subject is improved or the degree to which the progression of the symptom is suppressed, when the intervention is applied at the second time. More specifically, the intervention effect prediction unit 78 may output the following information as the second prediction information.

How close the angle around the target region is made to the normal value.

How close the size of the target region which has enlarged or shrunk is made to the normal value.

What percentage of wrinkles and spots on the target region are alleviated.

How long the condition of the target region is maintained.

How much walking ability (including stair climbing ability) is improved.

The second prediction information is the data of a type same as and/or similar to the first prediction information. For example, when the disorder is obesity, the second prediction information may be information regarding the weight, BMI, abdominal girth, visceral fat mass, blood pressure, blood glucose level, lipid, uric acid level or liver function level of the subject. For example, when the disorder is alopecia, the second prediction information may be information regarding the number of hairs, sex hormone level, Norwood scale, or Ludwig scale for the subject. For example, when the disorder is cataracts, the second prediction information may be information regarding visual acuity, vision breadth, the level of clouding of the eye lens, or Emery-Little scale for the subject. For example, when the disorder is periodontal disease, the second prediction information may be information regarding the degree of pain in tooth or gum, the degree of swelling of tooth or gum, the number of remaining teeth, gingival index, or periodontal pocket depth for the subject. For example, when the disorder is rheumatoid arthritis, the second prediction information may be information regarding the degree of pain, the degree of swelling, joint angle, joint movable range, Larsen scale, or Stein blocker scale for the whole body, upper limb, or lower limb of the subject. For example, when the disorder is Heberden's node, the second prediction information may be information regarding the degree of pain, the degree of swelling, or joint movable range for the hand of the subject. For example, when the disorder is hallux valgus, the second prediction information may be information regarding the degree of pain, the degree of swelling, joint movable range, HV angle, or M1-M2 angle for the feet of the subject. For example, when the disorder is osteoarthritis, the second prediction information may be information regarding the degree of pain, the degree of swelling, joint angle, joint movable range, or K-L scale for the whole body, upper limb, or lower limb of the subject. For example, when the disorder is spondylosis deformans, the second prediction information may be information regarding the degree of spinal curvature, spinal motion range, or K-L scale for the subject. For example, when the disorder is compression fracture, the second prediction information may be information regarding the degree of spinal curvature, spinal motion range, or K-L scale for the subject. For example, when the disorder is sarcopenia, the second prediction information may be information regarding the muscle mass of the subject.

### Intervention Information 85

The intervention information 85 is information regarding the intervention whose effect is estimated by the intervention effect prediction unit 78. The intervention information 85 of the intervention whose effect is estimated includes, for example, non-invasive treatment such as weight restriction, heat therapy, ultrasonic therapy, wearing of an orthotic device, or supplement intake. The intervention information 85 may include the estimated effect of invasive treatment such as surgical therapy.

### Training Processing on Neural Network Included in Prediction Image Generation Unit 72C

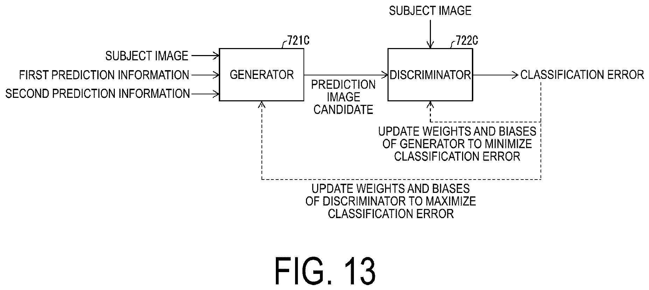

Hereinafter, with reference to FIG. 13, training processing will be described that is intended to generate a prediction image generation model to which a generative adversarial network (GAN) is applied. FIG. 13 is a diagram illustrating an example of configuration of the neural network included in the prediction image generation unit 72C. As illustrated in FIG. 13, the prediction image generation model to which the generative adversarial network is applied has two networks of a generator 721C and a discriminator 722C.

First, the first training unit 74 acquires a subject image and first prediction information from the storage 8C and inputs the subject image and the first prediction information to the generator 721C. The first training unit 74 inputs, to the generator 721C, second prediction information generated by the intervention effect prediction unit 78.

The generator 721C generates a prediction image candidate (false image) from the subject image, the first prediction information, and the second prediction information. The generator 721C may generate the prediction image candidate with reference to the real image included in the first training data set 82.

Then, the first training unit 74 inputs, to the discriminator 722C, prediction image candidates generated by the generator 721C and the real image included in the first training data set 82.

The discriminator 722C takes, as inputs, the real image from the first training data set 82 and the prediction image candidates generated by the generator 721C, and outputs, for each image, the probability that the image is real.

The first training unit 74 calculates a classification error indicating how correct the probability output by the discriminator 722C is. The first training unit 74 iteratively improves the discriminator 722C and the generator 721C using the error back-propagation method.

The first training unit 74 updates the weights and biases of the discriminator 722C and the weights and biases of the discriminator 722C until the probability output by the generator 721C satisfies a predetermined criterion. Thus, the prediction image generation unit 72C can generate a prediction image that is indistinguishable from the real image.

### Third Training Unit 79

The third training unit 79 controls training processing on the neural network included in the intervention effect prediction unit 78. The third teacher data 84 is used for this training.

Here, the third teacher data 84 is data used for machine learning for generating an intervention effect prediction model. The third teacher data 84 includes third training input data used as input data and third teaching data for calculating a difference from the first prediction information output by the intervention effect prediction unit 78.

The third training input data may include, for example, information indicating, for each of a plurality of patients to whom the intervention is applied, the timing when the intervention is applied, patient images each representing the target region of the respective patient, and symptom information regarding the onset or progression of the symptom of the target region at the time when the patient image of the patient is captured.

The third teaching data may include a patient image representing the target region of the patient at a time after the time when the patient image used for the third training input data is captured (for example, one year later), and symptom information regarding the onset or progression of the symptom at the target region of the patient. The third teaching data may include symptom information regarding the target region of each patient at the time when the patient image is taken. Here, the symptom information may include information regarding the onset of disorder of the patient or the progression of the symptom.

The third teacher data 84 may be information that includes condition information indicating the condition of the target region of each patient which information is acquired at a plurality of times in the past, and in which the condition information for each patient is associated with the intervention information indicating the intervention applied to each patient. That is, the third teacher data 84 may include effect information. The third teacher data 84 may be time series data in which patient images acquired at a plurality of times from a plurality of patients to whom the intervention is applied in the past are associated with information regarding the symptom of the target region at the time when the patient image is captured.

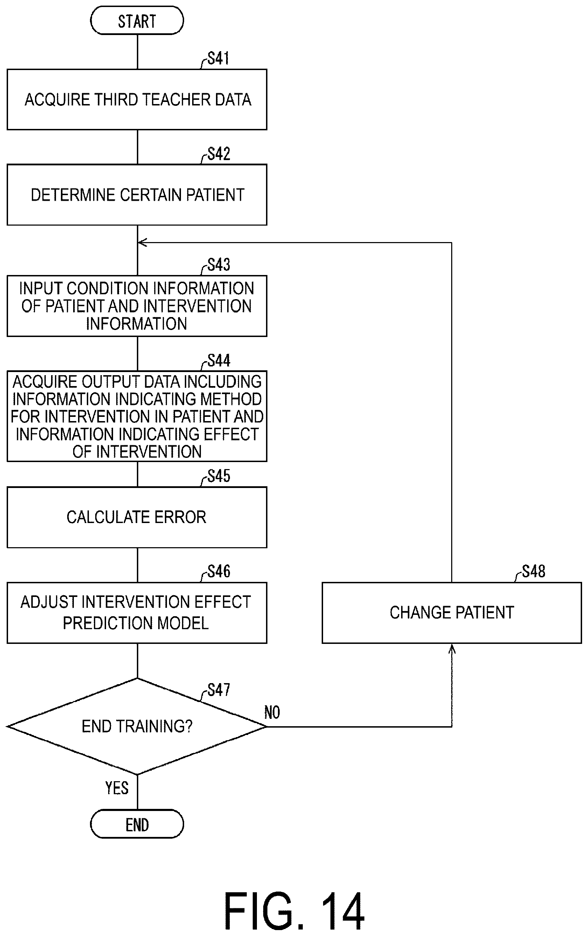

Training Processing on Intervention Effect Prediction Model Hereinafter, with reference to FIG. 7, the training processing for generating the intervention effect prediction model to which the neural network is applied will be described by using FIG. 14. FIG. 14 is a flowchart illustrating an example of the flow of the training processing on the neural network included in the intervention effect prediction unit 78.

First, the third training unit 79 acquires, from the storage 8C, the third training input data included in the third teacher data 84 (step S41). The third training input data includes, for example, (a) information indicating, for each of the plurality of patients to whom the intervention is applied, the timing when the intervention is applied, (b) pixel data of the patient image representing the target region of each patient, and (c) symptom information regarding the onset or progression of the symptom of the target region at the time when the patient image of the patient is captured.

Subsequently, the third training unit 79 determines a certain patient (step S42).

Subsequently, the third training unit 79 inputs, to the input layer 781, (a) information which may include, for the plurality of individual patients to whom the interventions are applied, information indicating when the interventions are applied, (b) pixel data of the patient image representing the target region of each patient, and (c) symptom information regarding the onset or progression of the symptom of the target region at the time when the patient image of the patient is captured, these pieces of information being included in the third training input data (step S43).

Then, the third training unit 79 acquires, from the output layer 782, output data corresponding to information indicating the method for intervention in a certain patient and/or the effect of the intervention (step S44). The output data includes the same contents as the third teaching data.

Subsequently, the third training unit 79 acquires the third teaching data included in the third teacher data 84. Then, the third training unit 79 compares the acquired output data with the information indicating the method for intervention in a certain patient and the effect of the intervention included in the third teaching data and calculates an error (step S45).

Then, the third training unit 79 adjusts the intervention effect prediction model to reduce the error (step S46).

Any known method is applicable to adjustment of the intervention effect prediction model. For example, the error back propagation method may be employed as a method for adjusting the intervention effect prediction model. The adjusted intervention effect prediction model is a new intervention effect prediction model, and in subsequent calculations, the intervention effect prediction unit 78 uses the new intervention effect prediction model. In the adjustment stage of the intervention effect prediction model, parameters used in the intervention effect prediction unit 78 may be adjusted.

The parameters include, for example, parameters used in the input layer 781 and the output layer 782. Specifically, the parameters include weighting factors used in the input layer 781 and the LSTM layers of the output layer 782. The parameters may include a filter coefficient.

When the error is not within the predetermined range, and the patient images of all the patients included in the third teacher data 84 are not input (NO in step S47), the third training unit 79 changes the patient (step S48), further returns to step S43, and repeats the training processing. When the error is within the predetermined range, and the patient images of all the patients included in the third teacher data 84 are already input (YES in step S47), the third training unit 79 ends the training processing.

### Third Teacher Data 84

The third teacher data 84 is data used for machine learning for generating an intervention effect prediction model. The third teacher data 84 may include information that includes condition information indicating the condition of the target region of each patient which information is acquired at a plurality of times in the past, and in which the condition information for each patient is associated with the intervention information indicating the intervention applied to each patient. That is, the third teacher data 84 may include effect information. The third teacher data 84 includes third training input data used as input data and third teaching data for calculating a difference from the first prediction information output by the intervention effect prediction unit 78.

The third training input data may include, for example, (a) information indicating, for a certain patient to whom the intervention is applied, the timing when the intervention is applied, (b) pixel data of the patient image representing the target region of the certain patient, and (c) symptom information regarding the onset or progression of the symptom of the target region at the time when the patient image of the certain patient is captured. The image data used as the third training input data may be medical image data representing the target region of each of a plurality of patients. The medical image data may include, for example, at least one selected from the group consisting of X-ray image data, CT image data, MRI image data, PET image data, and ultrasonic image data representing a captured image of the target region of each of the plurality of subjects.

The third teaching data may include a patient image representing the target region of the patient at a time after the time when the patient image used for the third training input data is captured (for example, one year later), and the condition information indicating the condition of the target region of the patient, and the symptom information regarding the target region. The third teaching data may be time series data in which patient images acquired at a plurality of times from a plurality of patients to whom the intervention is applied in the past are associated with information regarding the symptom of the joint at the time when the patient image is captured.

Specifically, the input data used during training of the intervention effect prediction unit 78 is (a) a subject image representing the target region of the subject at a certain time A that is included in the third training input data. Based on the input data described above, the intervention effect prediction unit 78 outputs, as the output data, first prediction information regarding the target region at a time B when a predetermined period (for example, three years) has elapsed from the time A. Specifically, the intervention effect prediction unit 78 outputs, as the output data, information indicating, for example, the angle around the target region of the subject at the time B, the degree of enlargement and shrinkage of the target region, the degree of wrinkles and spots on the target region, the timing when pain occurs in the target region and the degree of the pain, and the timing when invasive treatment is required for the target region. The output data indicated here are examples and are not limited thereto.

Specifically, the input data used during the training of the intervention effect prediction unit 78 is information indicating the onset of or the degree of progression of the disorder related to the target region of the patient at the certain time B included in the third teacher data 84 and information indicating the method for intervention included in the intervention information. Based on the input data as described above, the intervention effect prediction unit 78 outputs, as output data, information indicating the method for intervention in the subject and the effect of the intervention. Based on the input data described above, the intervention effect prediction unit 78 outputs, as output data, for example, information representing the degree to which the symptom of the disorder related to the target region of the patient is improved or the degree to which the progression of the symptom is suppressed, when the intervention is applied at the time B. More specifically, the intervention effect prediction unit 78 may output the above-described second prediction information as the output data.

### Processing Executed by Prediction System 100 or 100a

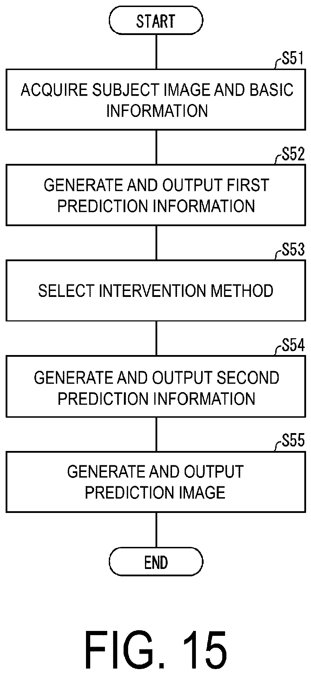

The flow of processing executed by the prediction system 100 or 100a will be described below with reference to FIG. 15. FIG. 15 is a flowchart illustrating the flow of processing executed by the prediction system 100 or 100a according to the present embodiment.

First, the prediction information acquirer 71 acquires a subject image. On the other hand, the basic information acquirer 76 acquires basic information (step S51: acquiring step).

Subsequently, in response to the input of the subject image and the basic information, the prediction information generation unit 75B generates first prediction information and outputs the first prediction information to the prediction information acquirer 71 and the intervention effect prediction unit 78 (step S52: first information prediction step).

Then, the intervention effect prediction unit 78 refers to the intervention information 85 and selects at least one of the methods for intervention included in the intervention information 85 (step S53: intervention method selection step).

Furthermore, in response to the input of the first prediction information, the intervention effect prediction unit 78 generates second prediction information for the selected method for intervention, and outputs the second prediction information to the prediction image generation unit 72C and the output controller 73C (step S54: intervention effect prediction step).

Subsequently, in response to the input of the subject image, the first prediction information, and the second prediction information, the prediction image generation unit 72C generates a prediction image and outputs the prediction image to the terminal device 2 (step S55: prediction image generation step).

The prediction system 100 or 100a can output the fact that the condition of the target region varies depending on the presence or absence of the effect of the intervention at the second time as a prediction image that is visually easy to understand. The prediction image is generated from the subject image which is an image of the subject in question, and thus the prediction image is a real image which is convincing to the subject. Therefore, for example, when a doctor or the like in charge of the subject presents the prediction image to the subject, the subject can be effectively made to understand the necessity of the intervention, enhancing the motivation of the subject for the intervention.

### Example of Software Implementation

A control block (in particular, the controller 7, 7A, 7B, 7C) of the prediction device 1, 1A, 1B, 1C may be implemented by a logic circuit (hardware) formed in an integrated circuit (IC chip) or the like or may be implemented by software.

In the latter case, the prediction device 1, 1A, 1B, 1C includes a computer that executes instructions of a program that is software that implements each function. The computer includes, for example, one or more processors and a computer-readable recording medium that stores the above program. Then, in the computer, the processor reads the above program from the recording medium and executes the read program to achieve the object of the present disclosure. As the processor, a central processing unit (CPU) can be used, for example. As the recording medium, a "non-transitory tangible medium", for example, a tape, a disk, a card, a semiconductor memory, and a programmable logic circuit can be used in addition to a read only memory (ROM). The computer may further include a random access memory (RAM) for loading the above program. The above program may be supplied to the computer via any transmission medium (communication network, broadcast wave, and the like) that can transmit the program. An aspect of the present disclosure may be implemented in the form of data signals embedded in a carrier wave in which the above program is embodied by electronic transmission.

In the present disclosure, the invention has been described above based on the various drawings and examples. However, the invention according to the present disclosure is not limited to each embodiment described above. That is, the invention according to the present disclosure can be modified in various ways within the scope illustrated in the present disclosure, and embodiments obtained by appropriately combining the technical means disclosed in different embodiments are also included in the technical scope of the invention according to the present disclosure. In other words, a person skilled in the art can easily make various variations or modifications based on the present disclosure. Note that these variations or modifications are included within the scope of the present disclosure.

### REFERENCE SIGNS

1, 1A, 1B, 1C Prediction device
2, 2a, 2b Terminal device
3, 3a, 3b First prediction information management device
4, 4a, 4b Subject image management device
5, 5a, 5b Medical facility
6 Communication network
7, 7A, 7B, 7C Controller
8, 8B, 8C Storage
9, 9a, 9b Electronic medical record management device
71 Prediction information acquirer
72, 72C Prediction image generation unit
73, 73C Output controller
74 First training unit
75 75B Prediction information generation unit
76 Basic information acquirer
77 Second training unit
78 Intervention effect prediction unit
79 Third training unit
81 Control program
82 First training data set
83 Second training data set
84 Third teacher data
85 Intervention information
100, 100a Prediction system
721, 721C Generator
722, 722C Discriminator
751, 781 Input layer
752, 782 Output layer

## Claims

1. A prediction system comprising:
a prediction information acquirer configured to acquire:
(a) a subject image representing a target region of a subject at a first time, and
(b) first prediction information regarding the target region at a second time when a predetermined period has elapsed from the first time; and
a prediction image generation unit configured to generate a prediction image from the first prediction information and the subject image by predicting a condition of the target region at the second time and output the prediction image.

2. The prediction system according to claim 1,
wherein the prediction image generation unit comprises a prediction image generation model configured to generate the prediction image by using the subject image and the first prediction information.

3. The prediction system according to claim 1 or 2,
wherein the prediction image comprises an image imitating at least a part of the subject image.

4. The prediction system according to any one of claims 1 to 3,
wherein the subject image comprises an appearance image representing the target region.

5. The prediction system according to any one of claims 1 to 4,
wherein the subject image comprises a medical image representing the target region.

6. The prediction system according to claim 5,
wherein the medical image comprises at least one selected from the group consisting of an X-ray image, a CT image, an MRI image, a PET image, and an ultrasonic image of the subj ect.

7. The prediction system according to any one of claims 1 to 6,
wherein the subject image comprises a captured image of any one of a whole body, a head, an upper body, a lower body, an upper limb, and a lower limb of the subject.

8. The prediction system according to any one of claims 1 to 7,
wherein the prediction image comprises an image obtained by predicting an effect, on the target region, of a disorder occurring in the target region.

9. The prediction system according to claim 8,
wherein the disorder comprises at least one selected from the group consisting of obesity, alopecia, cataracts, periodontal disease, rheumatoid arthritis, Heberden's node, hallux valgus, osteoarthritis, spondylosis deformans, compression fracture and sarcopenia.

10. The prediction system according to claim 2,
wherein the prediction image generation model comprises a neural network trained by using, as teacher data, a plurality pieces of image data each representing a target region.

11. The prediction system according to claim 2 or 10,
wherein the prediction image generation model comprises a generative adversarial network or an auto encoder.

12. The prediction system according to claim 8 or 9,
wherein the first prediction information comprises information regarding a shape and an appearance of the target region associated with the disorder of the target region.

13. The prediction system according to any one of claims 1 to 12, further comprising
a prediction information generation unit configured to generate the first prediction information from the subject image and output the first prediction information to the prediction information acquirer,
wherein the prediction information generation unit comprises a prediction information generation model configured to estimate the first prediction information from the subject image.

14. The prediction system according to claim 13, further comprising
a basic information acquirer configured to acquire basic information comprising at least one selected from the group consisting of a sex, an age, a height, a weight of the subject, and information indicating a condition of the target region of the subject at the first time,
wherein the prediction information generation model is configured to estimate the first prediction information from the subject image of the subject and the basic information of the subj ect.

15. The prediction system according to claim 13 or 14,
wherein the prediction information generation model comprises a neural network trained by using, as teacher data, patient information regarding patients each having a disorder of a target region, and
the patient information comprises information that comprises condition information indicating a condition of a target region of each of the patients acquired at a plurality of past times and where the condition information for each of the patients is associated with information indicating a time when the condition information is acquired.

16. The prediction system according to any one of claims 1 to 15, further comprising
an intervention effect prediction unit configured to output second prediction information indicating a method for intervention in the subject and an effect of the intervention by using the first prediction information as an input.

17. The prediction system of claim 16,
wherein the intervention effect prediction unit comprises, as an intervention effect prediction model, a neural network trained by using effect information as teacher data, and
the effect information comprises information that comprises condition information indicating a condition of a target region of each of the patients acquired at a plurality of past times and where the condition information for each of the patients is associated with intervention information indicating an intervention applied to each of the patients.

18. The prediction system according to claim 16 or 17,
wherein the method for the intervention comprises at least one selected from the group consisting of dietetic therapy, exercise therapy, drug therapy, orthotic therapy, rehabilitation, and surgical therapy.

19. A control method for a prediction system, the control method comprising:
acquiring:
(a) a subject image representing a target region of a subject at a first time, and
(b) first prediction information regarding the target region at a second time when a predetermined period has elapsed from the first time; and
generating a prediction image from the first prediction information and the subject image by predicting a condition of the target region at the second time and outputting the prediction image,
wherein the prediction system comprises a prediction image generation model configured to generate the prediction image by using the subject image and the first prediction information.

20. A control program for causing a computer to operate as the prediction system according to any one of claims 1 to 18, the control program causing the computer to operate as the prediction information acquirer and the prediction image generation unit.
